# EUROPEAN PATENT SPECIFICATION

(11) **EP 2 382 474 B1**
(45) Date of publication and mention of the grant of the patent: **04.03.2015**
(21) Application number: 10704115.4
(22) Date of filing: 18.01.2010
(51) Int. Cl.: G01N 33/574, G01N 33/68

(54) **SOLUBLE ICAM-1 AS BIOMARKER FOR PREDICTION OF THERAPEUTIC RESPONSE**
LÖSLICHES ICAM-1 ALS BIOMARKER ZUR VORHERSAGE EINER THERAPEUTISCHEN ANTWORT
ICAM-1 SOLUBLE EN TANT QUE BIOMARQUEUR POUR LA PRÉDICTION D'UNE RÉPONSE THÉRAPEUTIQUE

(30) Priority: 20.01.2009 EP 09305044; 07.04.2009 EP 09305294
(43) Date of publication of application: 02.11.2011
(73) Proprietor: Transgene SA, 67405 Illkirch Graffenstaden (FR)
(72) Inventor: ACRES, Bruce, 67000 Strasbourg (FR); MARIE-BASTIEN, Bérangère, 67230 Huttenheim (FR)
(74) Representative: Vossius & Partner
(86) International application number: PCT/EP2010/050532
(87) International publication number: WO 2010/084100

(56) References cited:
- DOWLATI A ET AL: "Cell adhesion molecules, vascular endothelial growth factor, and basic fibroblast growth factor in patients with non-small cell lung cancer treated with chemotherapy with or without bevacizumab--an Eastern Cooperative Oncology Group Study." CLIN CANCER RES, vol. 14, no. 5, 1 March 2008 (2008-03-01), pages 1407-1412, XP002575620 cited in the application
- MARANI T M ET AL: "Carboplatin-induced immune hemolytic anemia" TRANSFUSION, vol. 36, no. 11-12, 1996, pages 1016-1018, XP002575621
- ZOUMPOS I ET AL: "Urinary ICAM-1 levels can predict response in superficial bladder cancer treated with intravesical immunotherapy" EUR J CANCER. SUPPL, vol. 3, no. 2, 1 October 2005 (2005-10-01) , pages 250-251, XP005132985
- PASSALACQUA G ET AL: "The relationship between clinical efficacy of specific immunotherapy and serum intercellular adhesion molecule-1 levels" J INVEST ALLERGOL CLIN IMMUNOL, vol. 8, no. 2, March 1998 (1998-03), pages 123-124, XP009132441
- A M Jackson ET AL: "Changes in urinary cytokines and soluble intercellular adhesion molecule-1 (ICAM-1) in bladder cancer patients after Bacillus Calmette-Guerin (BCG) immunotherapy", Clin Exp Immunol, vol. 99, 1 March 1995 (1995-03-01), pages 369-375, XP055061187,

## Description

The present invention relates to the field of immunology and, in particular, to immunotherapy of a patient against diseases caused for example by infection or cancers. More particularly, the invention relates to methods for predicting whether a subject is or is not susceptible to developing a prophylactic or therapeutic response, preferably immune response, after such immunotherapy. The present invention relates to methods and compositions for improving the immune response raised *in vivo* by an immunogenic composition, in particular a vaccine.

Traditional vaccination techniques involving the introduction into an animal system of an antigen (e.g. peptides, proteins) which can induce an immune response, and thereby protect said animal against infection for example, have been known for many years. These techniques have further included the development of both live and inactivated vaccines. Live vaccines are typically attenuated non-pathogenic versions of an infectious agent that are capable of priming an immune response directed against a pathogenic version of the infectious agent.

In recent years there have been advances in the development of recombinant vaccines, especially recombinant live vaccines, in which foreign antigens of interest are encoded and expressed from a vector. Among them, vectors based on recombinant viruses have shown great promise and play an important role in the development of new vaccines. Many viruses have been investigated for their ability to express proteins from foreign pathogens or tumoral tissue, and to induce specific immunological responses against these antigens *in vivo.* Generally, these gene-based vaccines can stimulate potent humoral and cellular immune responses and viral vectors may be an effective strategy for both the delivery of antigen-encoding genes and the facilitation and enhancement of antigen presentation. In order to be utilized as a vaccine carrier, the ideal viral vector should be safe and enable efficient presentation of required pathogen-specific antigens to the immune system. Furthermore, the vector system must meet criteria that enable its production on a large-scale basis. Several viral vaccine vectors have thus emerged to date, all of them having relative advantages and limits depending on the proposed application (for a review on recombinant viral vaccines see for example Harrop and Carroll, 2006, Front Biosci., 11, 804-817 ; Yokoyama et al., 1997, J Vet Med Sci.,59, 311-322).

Following the observation in the early 1990's that plasmid DNA vectors could directly transfect animal cells in *vivo,* significant research efforts have also been undertaken to develop vaccination techniques based upon the use of DNA plasmids to induce immune response, by direct introduction into animals of DNA which encodes for antigens. Such techniques which are widely referred as DNA vaccination have now been used to elicit protective immune responses in large number of disease models. For a review on DNA vaccines, see Reyes-Sandoval and Ertl, 2001 (Current Molecular Medicine, 1, 217-243).

A general problem in vaccine field however has been the identification of a means of inducing a sufficiently strong immune response in vaccinated individuals to protect against infection and disease.

Therefore there has been for example major effort in recent years, to discover new drug compounds that act by stimulating certain key aspects of the immune system which will serve to increase the immune response induced by vaccines. Most of these compounds, referred as immune response modifiers (IRMs) or adjuvants, appear to act through basic immune system mechanisms via Toll-like receptors (TLRs) to induce various important cytokines biosynthesis (e.g., interferons, interleukins, tumor necrosis factor, etc. see for example Schiller et al., 2006, Exp Dermatol., 15, 331-341). Such compounds have been shown to stimulate a rapid release of certain dendritic cell, monocyte/macrophage-derived cytokines and are also capable of stimulating B cells to secrete antibodies which play an important role in the antiviral and antitumor activities of IRM compounds.

Alternatively, vaccination strategies have been proposed, most of them being based on a prime-boost vaccination regimen. According to these "prime-boost" vaccination protocols, the immune system is first induced by administering to the patient a priming composition and then boosted by administration of a boosting second composition (see for example EP1411974 or US20030191076).

Additionally, every member of a population may not be equally responsive to a particular treatment. For example, new compounds often fail in late clinical trials because of lack of efficacy in the population tested. While such compounds may not be effective in the overall population, there may be subpopulations sensitive to those failed compounds due to various reasons, including inherent differences in gene expression. By identifying a patient subpopulation sensitive to a compound, it is therefore possible to define method that can be used to rescue failed compounds. Subsequent-clinical trials restricted to a sensitive patient subpopulation may then demonstrate efficacy of a previously failed compound within that particular patient subpopulation, advancing the compound towards approval for use in that subpopulation. Accordingly, another way to improve immunotherapy efficacy, could be to determine patient population sensitive to the said therapy.

Intracellular adhesion molecule 1 (ICAM-1 or CD54) is an adhesion-related molecule belonging to the immunoglobulin superfamily. This molecule is found on the cell membrane of endothelial cells. When activated, ICAM-1 allows stable leukocyte adhesion to the endothelial surface. Initially, it has been shown that the primary structure of ICAM-1 has a typical hydrophobic transmembrane segment and is an insoluble molecule which is solubilized from cell membranes by lysing the cells in a non-ionic detergent (see EP0289949). Subsequent studies have identified an alternative molecular species of ICAM-1 designated sICAM-1 (or sCD54) which is soluble without the addition of a detergent (see US5821341). Soluble intercellular adhesion molecule-1 (sICAM-1) represents a circulating form of ICAM-1 that is constitutively expressed or is inducible on the cell surface of different cell lines. Soluble ICAM-1 has been found in such body fluids as serum, cerebrospinal fluid, synovial fluid, sputum, urine and bronchoalveolar lavage fluid. The release of soluble ICAM-1 is modulated by several cytokines and various factors (TNF alpha, interleukins, interferons, etc...). Although the role and functions of soluble ICAM-1 have not yet been completely elucidated, the evidence suggests its implication in some disease progression (e.g. atherosclerosis and inflammation, Hulthe et al, 2002, Clin Sci (Lond)., 103, 123-9). It has been proposed that at least its elevated levels may inform the clinician about pathological processes.

See for example :
US20060199239 discloses a method of assessing cardiac health in a patient, by measuring inflammation risk factors including level of ICAM-1 (disease level being defined above 285.2 ng/ml).
Molla et al. (1989, J Biol Chem., 264, 10589-94) suggest that levels of sICAM-1 are useful as indices of clinical manifestations of atherosclerosis.
Dong Soon Kim et al. (1999, Chest, 115, 1059-1065) suggest to use value of soluble ICAM-1 level as a marker of activity in sarcoidosis as in patients with active disease, the sICAM-1 levels in serum (575 ± 221 ng/mL) was higher than those for patients with inactive disease.
Demerath et al. (2001, Ann Hum Biol., 28, 664-78) have shown that the concentration of soluble sICAM-1 and sESEL, reflect the level of established CVD risk factors in apparently healthy men and women, adding to the evidence that these factors contribute to CVD through their inflammatory effects on the vascular endothelium.
See also Witkowska et al., 2004, Eur Cytokine Netw., 15, 91-98 which provides concentration of sICAM-1 in body fluids in various diseases.
Studies have shown that elevated levels of soluble ICAM and VCAM are related to disease activity in patients with various acute and chronic inflammatory diseases (Ilic et al, 2007, Med Pregl., 60 Suppl 2,128-32).
Dowlati et al., 2008, Clin. Cancer Res., 14, 1407-1412, have recently shown that ICAM levels were prognostic for survival and predictive of response to chemotherapy (i.e. carboplatin + paclitaxel).
Zoumpos et al., 2005, Eur. J. Cancer, Suppl. 3(2), 250-251 reported that urinary ICAM-1 levels can predict response in superficial bladder cancer treated with intravesical immunotherapy. Passalacqua et al., 1998, J. Invest. Allergol. Clin. Immunol. 8(2), 123-124 describe that the efficacy of specific immunotherapy can be monitored by measuring during the treatment the level of serum ICAM-1.

The present Invention relates to an ex-vivo method for testing whether a subject will respond therapeutically to a method of treatment comprising administration of an immunogenic composition, wherein the testing method comprises the step of measuring levels of sICAM-1 in a blood sample obtained from said patient before administration of said immunogenic composition, wherein low levels of sICAM-1 indicates that the subject will develop a prophylactic or therapeutic response, preferably a prophylactic or therapeutic immune response, towards the immunogenic composition with low levels of sICAM-1 being fewer than 300 ng/ml, and wherein said immunogenic composition contains all or part of at least one targeted antigen or at least one recombinant vector expressing in vivo all or part of at least one heterologous nucleotide sequence encoding all or part of at least one targeted antigen.
According to one embodiment, the method of treatment is a method of treating cancer.

As used herein throughout the entire application, the terms "a" and "an" are used in the sense that they mean "at least one", "at least a first", "one or more" or "a plurality" of the referenced compounds or steps, unless the context dictates otherwise. For example, the term "a cell" includes a plurality of cells including a mixture thereof. More specifically, "at least one" and "one or more" means a number which is one or greater than one, with a special preference for one, two or three.

The term "and/or" wherever used herein includes the meaning of "and", "or" and "all or any other combination of the elements connected by said term".

The term "about" or "approximately" as used herein means within 20%, preferably within 10%, and more preferably within 5%.

The terms "patient", "subject" refer to a vertebrate, particularly a member of the mammalian species and includes, but is not limited to, domestic animals, sport animals, primates including humans.

According to special embodiments, the terms "a patient will or is susceptible to respond therapeutically" mean that in said patient, after the administration of an immunogenic composition, an immune response is raised (i.e. the raised immune response). According to preferred embodiments, the terms "a patient will or is susceptible to respond therapeutically" mean that the said patient has an increase of survival rate (see example section).

According to the Invention, an increase of survival rate is observed by comparing the treatment of the present Invention with the reference treatment, i.e. without patient selection based on sICAM-1 levels. In preferred embodiment, an increase of survival rate is observed when the treated patient is still alive after 5 years, 2 years, 12 months, 8 months, 6 months, 1 month post treatment according to the Invention.

As used herein, the term "treatment" or "treating" encompasses prophylaxis and/or therapy. Accordingly the immunogenic combinations or methods of the present invention are not limited to therapeutic applications and can be used in prophylaxis ones. This is covered by the term "to developing a prophylactic or therapeutic response, preferably immune response," herein. "Prophylaxis" is not limited to preventing immediate diseases (e.g. infectious diseases), it further encompasses prevention of long term consequences of these infections such as cirrhosis or cancer.

An "effective amount" or a "sufficient amount" of an active compound is an amount sufficient to effect beneficial or desired results, including clinical results. An effective amount can be administered in one or more administrations. A "therapeutically effective amount" is an amount to effect beneficial clinical results, including, but not limited to, alleviation of one or more symptoms associated with tumor progression, viral infection as well as prevention of disease (e.g. prevention of one or more symptoms of cancer or infection), stimulating/inducing/increasing a subject's immune system to ameliorate a current condition or to protect against or to reduce present or future harm or infections (including viral, bacterial, parasitic infections), e.g., reduced tumour cell proliferation or survival, reduced pathogen replication or spread in a subject or a detectably reduced unwanted symptom(s) associated with a condition, extend patient survival.

As used herein, the terms "sICAM-1" or sCD54 means soluble intercellular adhesion molecule-1 and represents a circulating form of ICAM-1. sICAM-1 or sCD54 can be used interchangeably.

According to the Invention, the levels of sICAM-1 can be determined by any method known in the art, for example the materials and methods of the present invention may be used with Luminex technology (Luminex Corporation, Austin, Tex.) or enzyme-linked immunosorbant assays (ELISA, numerous ELISA kits are commercially available e.g. by CliniScience, Diaclone, Biosource).

According to the Invention, the levels of sICAM-1 can be determined on total blood sample or on plasma or serum. Accordingly, "obtaining a blood sample from the subject" should be understood as including further treatment of the said blood sample if sICAM-1 level has to be measured in plasma or serum.

According to one embodiment of the Invention, the level of sICAM-1 is determined by using antibodies.

According to one specific embodiment of the Invention, said antibodies are monoclonal antibodies.

According to one specific embodiment of the Invention, said antibodies are tagged for example by fluorescence, radiolabel, enzyme, biotin, or any other methods designed to render sICAM-1 labelled with said antibodies detectable. These techniques are widely used and known in the art.

Anti-CD54 antibodies, especially monoclonal antibodies are widely available in the commerce. See for example antibody 3H1547 from Anticorps-enligne.fr, antibody 5540-P (Biocytex).

The term "low levels of sICAM-1" means levels fewer than about 300 ng/ml, preferably fewer than about 250 ng/ml, more preferably fewer than about 224 ng/ml and even preferably fewer than about 200 ng/ml. Said level can be measured by Multi-analyte plasma protein profiling using the Luminex® system (e.g.Luminex® xMAP™ technology, R&D Systems).

As used herein, the terms "immunogenic composition" "vaccine composition", "vaccine" or similar terms can be used interchangeably and mean an agent suitable for stimulating/inducing/increasing a subject's immune system to ameliorate a current condition or to protect against or to reduce present or future harm or infections (including viral, bacterial, parasitic infections), e.g., reduced tumour cell proliferation or survival, reduced pathogen replication or spread in a subject or a detectably reduced unwanted symptom(s) associated with a condition, extend patient survival. Said immunogenic composition can contain (i) all or part of at least one targeted antigen and/or (ii) at least one recombinant vector expressing *in vivo* all or part of at least one heterologous nucleotide sequence, especially an heterologous nucleotide sequence encoding all or part of at least one targeted antigen. According to a preferred embodiment, the immunogenic composition of the Invention further comprises (iii) at least one immune response modifier in combination with (i) and/or (ii). Examples of such immune response modifiers (IRMs), include the CpG oligonucleotides (see US 6,194,388; US2006094683; WO 2004039829 for example), lipopolysaccharides, polyinosic:polycytidylic acid complexes (Kadowaki, et al., 2001, J. Immunol. 166, 2291-2295), and polypeptides and proteins known to induce cytokine production from dendritic cells and/or monocyte/macrophages. Other examples of such immune response modifiers (IRMs) are small organic molecule such as imidazoquinolinamines, imidazopyridine amines, 6,7-fused cycloalkylimidazopyridine amines, imidazonaphthyridine amines, oxazoloquinoline amines, thiazoloquinoline amines and 1,2-bridged imidazoquinoline amines (see for example US 4,689,338; US 5,389,640; US 6,110,929; and US 6,331,539).

As used herein, the term "antigen" refers to any substance, including complex antigen (e.g. tumour cells, virus infected cells, etc...), that is capable of being the target of an immune response. An antigen may be the target of, for example, a cell-mediated and/or humoral immune response raised by a patient. The term "antigen" encompasses for example all or part of viral antigens, tumour-specific or tumour-related antigens, bacterial antigens, parasitic antigens, allergens and the like:
- Viral antigens include for example antigens from hepatitis viruses A, B, C, D and E, HIV, herpes viruses, cytomegalovirus, varicella zoster, papilloma viruses, Epstein Barr virus, influenza viruses, para-influenza viruses, adenoviruses, coxsakie viruses, picorna viruses, rotaviruses, respiratory syncytial viruses, pox viruses, rhinoviruses, rubella virus, papovirus, mumps virus, measles virus; some non-limiting examples of known viral antigens include the following : antigens derived from HIV-1 such as tat, nef, gp120 or gp160, gp40, p24, gag, env, vif, vpr, vpu, rev or part and/or combinations thereof; antigens derived from human herpes viruses such as gH, gL gM gB gC gK gE or gD or part and/or combinations thereof or Immediate Early protein such asICP27, ICP47, ICP4, ICP36 from HSV1 or HSV2 ; antigens derived from cytomegalovirus, especially human cytomegalovirus such as gB or derivatives thereof ; antigens derived from Epstein Barr virus such as gp350 or derivatives thereof; antigens derived from Varicella Zoster Virus such as gpl, 11, 111 and IE63; antigens derived from a hepatitis virus such as hepatitis B , hepatitis C or hepatitis E virus antigen (e.g. env protein E1 or E2, core protein, NS2, NS3, NS4a, NS4b, NS5a, NS5b, p7, or part and/or combinations thereof of HCV) ; antigens derived from human papilloma viruses (for example HPV6,11,16,18, e.g. L1, L2, E1, E2, E3, E4, E5, E6, E7, or part and/or combinations thereof) ; antigens derived from other viral pathogens, such as Respiratory Syncytial virus (e.g. F and G proteins or derivatives thereof), parainfluenza virus, measles virus, mumps virus, flaviviruses (e. g. Yellow Fever Virus, Dengue Virus, Tick-borne encephalitis virus, Japanese Encephalitis Virus) or Influenza virus cells (e.g. HA, NP, NA, or M proteins, or part and/or combinations thereof);
- tumor-specific or -related antigens include but are not limited to, carcinoma, lymphoma, blastoma, sarcoma, and leukemia. More particular examples of such cancers include breast cancer, prostate cancer, colon cancer, squamous cell cancer, small-cell lung cancer, non-small cell lung cancer, gastrointestinal cancer, pancreatic cancer, glioblastoma, cervical cancer, ovarian cancer, liver cancer, bladder cancer, hepatoma, colorectal cancer, endometrial carcinoma, salivary gland carcinoma, kidney cancer, liver cancer, vulval cancer, thyroid cancer, hepatic carcinoma and various types of head and neck cancer, renal cancer, malignant melanoma, laryngeal cancer, prostate cancer. Cancer antigens are antigens which can potentially stimulate apparently tumor-specific immune responses. Some of these antigens are encoded, although not necessarily expressed, by normal cells. These antigens can be characterized as those which are normally silent (i.e., not expressed) in normal cells, those that are expressed only at low levels or at certain stages of differentiation and those that are temporally expressed such as embryonic and fetal antigens. Other cancer antigens are encoded by mutant cellular genes, such as oncogenes (e.g., activated ras oncogene), suppressor genes (e.g., mutant p53), fusion proteins resulting from internal deletions or chromosomal translocations. Still other cancer antigens can be encoded by viral genes such as those carried on RNA and DNA tumor viruses. Some non-limiting examples of tumor-specific or -related antigens include MART-1/Melan-A, gp100, Dipeptidyl peptidase IV (DPPIV), adenosine deaminase-binding protein (ADAbp), cyclophilin b, Colorectal associated antigen (CRC)-C017-1A/GA733, Carcinoembryonic Antigen (CEA) and its immunogenic epitopes CAP-1 and CAP-2, etv6, aml1, Prostate Specific Antigen (PSA) and its immunogenic epitopes PSA-1, PSA-2, and PSA-3, prostate-specific membrane antigen (PSMA), T-cell receptor/CD3-zeta chain, MAGE-family of tumor antigens (e.g., MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4, MAGE-C5), GAGE-family of tumor antigens (e.g., GAGE-1, GAGE-2, GAGE-3, GAGE-4, GAGE-5, GAGE-6, GAGE-7, GAGE-8, GAGE-9), BAGE, RAGE, LAGE-1, NAG, GnT-V, MUM-1, CDK4, tyrosinase, p53, MUC family (e.g. MUC-1), HER2/neu, p21ras, RCAS1, alpha-fetoprotein, E-cadherin, alpha-catenin, beta-catenin and gamma-catenin, p120ctn, gp100.sup.Pmel117, PRAME, NY-ESO-1, cdc27, adenomatous polyposis coli protein (APC), fodrin, Connexin 37, Ig-idiotype, p15, gp75, GM2 and GD2 gangliosides, viral products such as human papilloma virus proteins, Smad family of tumor antigens, lmp-1, P1A, EBV-encoded nuclear antigen (EBNA)-1, brain glycogen phosphorylase, SSX-1, SSX-2 (HOM-MEL-40), SSX-1, SSX-4, SSX-5, SCP-1 and CT-7, and c-erbB-2;
- bacterial antigens includes for example antigens from Mycobacteria causing TB and leprosy, pneumocci, aerobic gram negative bacilli, mycoplasma, staphyloccocal infections, streptococcal infections, salmonellae, chlamydiae, neisseriae;
- other antigens includes for example antigens from malaria, leishmaniasis, trypanosomiasis, toxoplasmosis, schistosomiasis, filariasis;
- allergens refer to a substance that can induce an allergic or asthmatic response in a susceptible subject. The list of allergens is enormous and can include pollens, insect venoms, animal dander dust, fungal spores and drugs (e.g. penicillin). Examples of natural, animal and plant allergens include but are not limited to proteins specific to the following genuses: Canine (Canis familiaris); Dermatophagoides (e.g. Dermatophagoides farinae); Felis (Felis domesticus); Ambrosia (Ambrosia artemiisfolia; Lolium (e.g. Lolium perenne or Lolium multiflorum); Cryptomeria (Cryptomeria japonica); Alternaria (Alternaria alternata); Alder; Alnus (Alnus gultinoasa); Betula (Betula verrucosa); Quercus (Quercus alba); Olea (Olea europa); Artemisia (Artemisia vulgaris); Plantago (e.g. Plantago lanceolata); Parietaria (e.g. Parietaria officinalis or Parietaria judaica); Blattella (e.g. Blattella germanica); Apis (e.g. Apis multiflorum); Cupressus (e.g. Cupressus sempervirens, Cupressus arizonica and Cupressus macrocarpa); Juniperus (e.g. Juniperus sabinoides, Juniperus virginiana, Juniperus communis and Juniperus ashei); Thuya (e.g. Thuya orientalis); Chamaecyparis (e.g. Chamaecyparis obtusa); Periplaneta (e.g. Periplaneta americana); Agropyron (e.g. Agropyron repens); Secale (e.g. Secale cereale); Triticum (e.g. Triticum aestivum); Dactylis (e.g. Dactylis glomerata); Festuca (e.g. Festuca elatior); Poa (e.g. Poa pratensis or Poa compressa); Avena (e.g. Avena sativa); Holcus (e.g. Holcus lanatus); Anthoxanthum (e.g. Anthoxanthum odoratum); Arrhenatherum (e.g. Arrhenatherum elatius); Agrostis (e.g. Agrostis alba); Phleum (e.g. Phleum pratense); Phalaris (e.g. Phalaris arundinacea); Paspalum (e.g. Paspalum notatum); Sorghum (e.g. Sorghum halepensis); and Bromus (e.g. Bromus inermis).

According to one embodiment, said antigen is encoded by an heterologous nucleotide sequence and is expressed *in vivo* by a recombinant vector.

In a particularly preferred embodiment the heterologous nucleotide sequence of the present invention, encodes one or more of all or part of the following antigens HBV-PreS1 PreS2 and Surface env proteins, core and polHIV-gp120 gp40, gp160, p24, gag, pol, env, vif, vpr, vpu, tat, rev, nef; HPV-E1, E2, E3, E4, E5, E6, E7, E8, L1, L2 (see for example WO 90/10459, WO 98/04705, WO 99/03885); HCV env protein E1 or E2, core protein, NS2, NS3, NS4a, NS4b, NS5a, NS5b, p7 (see for example WO2004111082, WO2005051420); Muc-1 (see for example US 5,861,381; US6,054,438; WO98/04727; WO98/37095).

According to variants of the invention, the immunogenic composition contains at least two antigens, or an heterologous nucleotide sequence encoding at least two antigens, or at least two heterologous nucleotide sequences encoding at least two antigens, or any combination thereof.

According to another special embodiment, said heterologous nucleotide sequence of the present invention, encodes all or part of HPV antigen (s) selected in the group consisting of E6 early coding region of HPV, E7 early coding region of HPV and derivates or combination thereof.

The HPV antigen encoded by the recombinant vector according to the invention is selected in the group consisting of an HPV E6 polypeptide, an HPV E7 polypeptide or both an HPV E6 polypeptide and an HPV E7 polypeptide. The present invention encompasses the use of any HPV E6 polypeptide which binding to p53 is altered or at least significantly reduced and/or the use of any HPV E7 polypeptide which binding to Rb is altered or at least significantly reduced (Munger et al., 1989, EMBO J. 8, 4099-4105; Crook et al., 1991, Cell 67, 547-556; Heck et al., 1992, Proc. Natl. Acad. Sci. USA 89, 4442-4446; Phelps et al., 1992, J. Virol. 66, 2148-2427). A non-oncogenic HPV-16 E6 variant which is suitable for the purpose of the present invention is deleted of one or more amino acid residues located from approximately position 118 to approximately position 122 (+1 representing the first methionine residue of the native HPV-16 E6 polypeptide), with a special preference for the complete deletion of residues 118 to 122 (CPEEK). A non-oncogenic HPV-16 E7 variant which is suitable for the purpose of the present invention is deleted of one or more amino acid residues located from approximately position 21 to approximately position 26 (+1 representing the first amino acid of the native HPV-16 E7 polypeptide, with a special preference for the complete deletion of residues 21 to 26 (DLYCYE). According to a preferred embodiment, the one or more HPV-16 early polypeptide(s) in use in the invention is/are further modified so as to improve MHC class I and/or MHC class II presentation, and/or to stimulate anti-HPV immunity. HPV E6 and E7 polypeptides are nuclear proteins and it has been previously shown that membrane presentation permits to improve their therapeutic efficacy (see for example WO99/03885). Thus, it may be advisable to modify at least one of the HPV early polypeptide(s) so as to be anchored to the cell membrane. Membrane anchorage can be easily achieved by incorporating in the HPV early polypeptide a membrane-anchoring sequence and if the native polypeptide lacks it a secretory sequence (i.e. a signal peptide). Membrane-anchoring and secretory sequences are known in the art. Briefly, secretory sequences are present at the N-terminus of the membrane presented or secreted polypeptides and initiate their passage into the endoplasmic reticulum (ER). They usually comprise 15 to 35 essentially hydrophobic amino acids which are then removed by a specific ER-located endopeptidase to give the mature polypeptide. Membrane-anchoring sequences are usually highly hydrophobic in nature and serves to anchor the polypeptides in the cell membrane (see for example Branden and Tooze, 1991, in Introduction to Protein Structure p. 202-214, NY Garland).

The choice of the membrane-anchoring and secretory sequences which can be used in the context of the present invention is vast. They may be obtained from any membrane-anchored and/or secreted polypeptide comprising it (e.g. cellular or viral polypeptides) such as the rabies glycoprotein, of the HIV virus envelope glycoprotein or of the measles virus F protein or may be synthetic. The membrane anchoring and/or secretory sequences inserted in each of the early HPV-16 polypeptides used according to the invention may have a common or different origin. The preferred site of insertion of the secretory sequence is the N-terminus downstream of the codon for initiation of translation and that of the membrane-anchoring sequence is the C-terminus, for example immediately upstream of the stop codon.

The HPV E6 polypeptide in use in the present invention is preferably modified by insertion of the secretory and membrane-anchoring signals of the measles F protein. Optionally or in combination, the HPV E7 polypeptide in use in the present invention is preferably modified by insertion of the secretory and membrane-anchoring signals of the rabies glycoprotein.

The therapeutic efficacy of the recombinant vector can also be improved by using one or more nucleic acid encoding immunopotentiator polypeptide(s). For example, it may be advantageous to link the HPV early polypeptide(s) to a polypeptide such as calreticulin (Cheng et al., 2001, J. Clin. Invest. 108, 669-678), Mycobacterium tuberculosis heat shock protein 70 (HSP70) (Chen et al., 2000, Cancer Res. 60, 1035-1042), ubiquitin (Rodriguez et al., 1997, J. Virol. 71, 8497-8503) or the translocation domain of a bacterial toxin such as Pseudomonas aeruginosa exotoxin A (ETA(dIII)) (Hung et al., 2001 Cancer Res. 61, 3698-3703).

According to another embodiment, the recombinant vector according to the invention comprises a nucleic acid encoding one or more early polypeptide(s) as above defined, and more particularly HPV-16 and/or HPV-18 early E6 and/or E7 polypeptides.

According to another special and preferred embodiment, said heterologous nucleotide sequence of the present invention, encodes all or part of MUC 1 antigen or derivates thereof.

According to another special embodiment, said heterologous nucleotide sequence of the present invention, encodes one or more of all or part of the followings: HCV env protein E1 or E2, core protein, NS2, NS3, NS4a, NS4b, NS5a, NS5b, p7 or derivates thereof. According to another special embodiment, said heterologous nucleotide sequence of the present invention, encodes one or more fusion protein wherein the configuration is not native in the sense that at least one of the NS polypeptides appears in an order which is distinct from that of the native configuration. Thus, if the fusion protein comprises a NS3 polypeptide, a NS4A polypeptide and a NS5B polypeptide, the native configuration would be NS3-NS4A-NS5B with NS3 at the N-terminus and NS5B at the C-terminus. In contrast, a non-native configuration can be NS5B-NS3-NS4A, NS5B-NS4A-NS3, NS4A-NS3-NS5B, NS4A-NS5B-NS3 or NS3-NS5B-NS4A. In particular, the fusion protein according to the invention comprises at least one of the followings:
∘ A NS4A polypeptide fused directly or through a linker to the N-terminus of a NS3 polypeptide;
∘ A NS3 polypeptide fused directly or through a linker to the N-terminus of a NS5B polypeptide;
∘ A NS4B polypeptide fused directly or through a linker to the N-terminus of a NS5B polypeptide;
∘ A NS4A polypeptide fused directly or through a linker to the N-terminus of a NS3 polypeptide which is fused directly or through a linker to the N-terminus of a NS4B polypeptide; and/or
∘ A NS3 polypeptide fused directly or through a linker to the N-terminus of a NS4B polypeptide which is fused directly or through a linker to the N-terminus of a NS5B polypeptide.

In such specific portions of the fusion protein of the invention, each of the NS polypeptides can be independently native or modified. For example, the NS4A polypeptide included in the NS4A-NS3 portion can be native whereas the NS3 polypeptide comprises at least one of the modifications described below.

If needed, the nucleic acid molecule in use in the invention may be optimized for providing high level expression of the targeted antigen (e.g. HPV early polypeptide(s)) in a particular host cell or organism, e.g. a human host cell or organism. Typically, codon optimisation is performed by replacing one or more "native" (e.g. HPV) codon corresponding to a codon infrequently used in the mammalian host cell by one or more codon encoding the same amino acid which is more frequently used. This can be achieved by conventional mutagenesis or by chemical synthetic techniques (e.g. resulting in a synthetic nucleic acid). It is not necessary to replace all native codons corresponding to infrequently used codons since increased expression can be achieved even with partial replacement. Moreover, some deviations from strict adherence to optimised codon usage may be made to accommodate the introduction of restriction site(s).

As used herein, the term "recombinant vector" refers to viral as well as non viral vectors, including extrachromosomal (e.g. episome), multicopy and integrating vectors (i.e. for being incorporated into the host chromosomes). Particularly important in the context of the invention are vectors for use in gene therapy (i.e. which are capable of delivering the nucleic acid to a host organism) as well as expression vectors for use in various expression systems. Suitable non viral vectors include plasmids such as pREP4, pCEP4 (Invitrogene), pCI (Promega), pCDM8 (Seed, 1987, Nature 329, 840), pVAX and pgWiz (Gene Therapy System Inc; Himoudi et al., 2002, J. Virol. 76, 12735-12746). Suitable viral vectors may be derived from a variety of different viruses (e.g. retrovirus, adenovirus, AAV, poxvirus, herpes virus, measle virus, foamy virus and the like). As used herein, the term "viral vector" encompasses vector DNA/RNA as well as viral particles generated thereof. Viral vectors can be replication-competent, or can be genetically disabled so as to be replication-defective or replication-impaired. The term "replication-competent" as used herein encompasses replication-selective and conditionally-replicative viral vectors which are engineered to replicate better or selectively in specific host cells (e.g. tumoral cells).

In one aspect, the recombinant vector in use in the invention is a recombinant adenoviral vector (for a review, see "Adenoviral vectors for gene therapy", 2002, Ed D. Curiel and J. Douglas, Academic Press). It can be derived from a variety of human or animal sources and any serotype can be employed from the adenovirus serotypes 1 through 51. Particularly preferred are human adenoviruses 2 (Ad2), 5 (Ad5), 6 (Ad6), 11 (Ad11) 24 (Ad24) and 35 (Ad35). Such adenovirus are available from the American Type Culture Collection (ATCC, Rockville, Md.), and have been the subject of numerous publications describing their sequence, organization and methods of producing, allowing the artisan to apply them (see for example US 6,133,028; US 6,110,735; WO 02/40665; WO 00/50573; EP 1016711; Vogels et al., 2003, J. Virol. 77, 8263-8271).

The adenoviral vector in use in the present invention can be replication-competent. Numerous examples of replication-competent adenoviral vectors are readily available to those skill in the art (see, for example, Hernandez-Alcoceba et al., 2000, Human Gene Ther. 11, 2009-2024; Nemunaitis et al., 2001, Gene Ther. 8, 746-759; Alemany et al., 2000, Nature Biotechnology 18, 723-727). For example, they can be engineered from a wild-type adenovirus genome by deletion in the E1A CR2 domain (see for example WO00/24408) and/or by replacement of the native E1 and/or E4 promoters with tissue, tumor or cell status-specific promoters (see for example US 5,998,205, WO99/25860, US 5,698,443, WO00/46355, WO00/15820 and WO01/36650).

Alternatively, the adenoviral vector in use in the invention is replication-defective (see for example WO94/28152; Lusky et al., 1998, J. Virol 72, 2022-2032). Preferred replication-defective adenoviral vectors are E1-defective (see for example US 6,136,594 and US 6,013,638), with an E1 deletion extending from approximately positions 459 to 3328 or from approximately positions 459 to 3510 (by reference to the sequence of the human adenovirus type 5 disclosed in the GeneBank under the accession number M 73260 and in Chroboczek et al., 1992, Virol. 186, 280-285). The cloning capacity can further be improved by deleting additional portion(s) of the adenoviral genome (all or part of the non essential E3 region or of other essential E2, E4 regions). Insertion of a nucleic acid in any location of the adenoviral vector can be performed through homologous recombination as described in Chartier et al. (1996, J. Virol. 70, 4805-4810). For example, the nucleic acid encoding the HPV-16 E6 polypeptide can be inserted in replacement of the E1 region and the nucleic acid encoding the HPV-16 E7 polypeptide in replacement of the E3 region or vice *versa.*

In another and preferred aspect, the vector in use in the invention is a poxviral vector (see for example Cox et al. in "Viruses in Human Gene Therapy" Ed J. M. Hos, Carolina Academic Press). According to another preferred embodiment it is selected in the group consisting of vaccinia virus, suitable vaccinia viruses include without limitation the Copenhagen strain (Goebel et al., 1990, Virol. 179, 247-266 and 517-563; Johnson et al., 1993, Virol. 196, 381-401), the Wyeth strain and the highly attenuated attenuated virus derived thereof including MVA (for review see Mayr, A., et al., 1975, Infection 3, 6-14) and derivates thereof (such as MVA vaccinia strain 575 (ECACC V00120707 - US 6,913,752), NYVAC (see WO 92/15672 - Tartaglia et al., 1992, Virology, 188, 217-232). Determination of the complete sequence of the MVA genome and comparison with the Copenhagen VV genome has allowed the precise identification of the seven deletions (I to VII) which occurred in the MVA genome (Antoine et al., 1998, Virology 244, 365-396), any of which can be used to insert the antigen-encoding nucleic acid. The vector may also be obtained from any other member of the poxviridae, in particular fowlpox (e.g. TROVAC, see Paoletti et al, 1995, Dev Biol Stand., 84, 159-163); canarypox (e.g. ALVAC, WO 95/27780, Paoletti et al, 1995, Dev Biol Stand., 84, 159-163); pigeonpox; swinepox and the like. By way of example, persons skilled in the art may refer to WO 92 15672 (incorporated by reference) which describes the production of expression vectors based on poxviruses capable of expressing such heterologous nucleotide sequence, especially nucleotide sequence encoding antigen.

The basic technique for inserting the nucleic acid and associated regulatory elements required for expression in a poxviral genome is described in numerous documents accessible to the man skilled in the art (Paul et al., 2002, Cancer gene Ther. 9, 470-477; Piccini et al., 1987, Methods of Enzymology 153, 545-563 ; US 4,769,330 ; US 4,772,848 ; US 4,603,112 ; US 5,100,587 and US 5,179,993). Usually, one proceed through homologous recombination between overlapping sequences (i.e. desired insertion site) present both in the viral genome and a plasmid carrying the nucleic acid to insert.

The nucleic acid encoding the antigen of the Invention is preferably inserted in a nonessential locus of the poxviral genome, in order that the recombinant poxvirus remains viable and infectious. Nonessential regions are non-coding intergenic regions or any gene for which inactivation or deletion does not significantly impair viral growth, replication or infection. One may also envisage insertion in an essential viral locus provided that the defective function is supplied *in trans* during production of viral particles, for example by using an helper cell line carrying the complementing sequences corresponding to those deleted in the poxviral genome.

When using the Copenhagen vaccinia virus, the antigen-encoding nucleic acid is preferably inserted in the thymidine kinase gene (tk) (Hruby et al., 1983, Proc. Natl. Acad. Sci USA 80, 3411-3415 ; Weir et al., 1983, J. Virol. 46, 530-537). However, other insertion sites are also appropriate, e.g. in the hemagglutinin gene (Guo et al., 1989, J. Virol. 63, 4189-4198), in the K1L locus, in the u gene (Zhou et al., 1990, J. Gen. Virol. 71, 2185-2190) or at the left end of the vaccinia virus genome where a variety of spontaneous or engineered deletions have been reported in the literature (Altenburger et al., 1989, Archives Virol. 105, 15-27 ; Moss et al. 1981, J. Virol. 40, 387-395 ; Panicali et al., 1981, J. Virol. 37, 1000-1010 ; Perkus et al, 1989, J. Virol. 63, 3829-3836 ; Perkus et al, 1990, Virol. 179, 276-286 ; Perkus et al, 1991, Virol. 180, 406-410).

When using MVA, the antigen-encoding nucleic acid can be inserted in any one of the identified deletions I to VII as well as in the D4R locus, but insertion in deletion II or III is preferred (Meyer et al., 1991, J. Gen. Virol. 72, 1031-1038 ; Sutter et al., 1994, Vaccine 12, 1032-1040).

When using fowlpox virus, although insertion within the thymidine kinase gene may be considered, the antigen-encoding nucleic acid is preferably introduced in the intergenic region situated between ORFs 7 and 9 (see for example EP 314 569 and US 5,180,675).

According to one special embodiment, said recombinant vector is a recombinant plasmid DNA or a recombinant viral vector.

According to another special embodiment, said recombinant viral vector is a recombinant adenoviral vector.

According to another special embodiment, said recombinant viral vector is a recombinant vaccinia vector.

According to one preferred embodiment, said recombinant vaccinia vector is a recombinant MVA vector.

Preferably, the antigen-encoding nucleic acid in use in the invention is in a form suitable for its expression in a host cell or organism, which means that the nucleic acid sequence encoding the antigen are placed under the control of one or more regulatory sequences necessary for its expression in the host cell or organism. As used herein, the term "regulatory sequence" refers to any sequence that allows, contributes or modulates the expression of a nucleic acid in a given host cell, including replication, duplication, transcription, splicing, translation, stability and/or transport of the nucleic acid or one of its derivative (i.e. mRNA) into the host cell. It will be appreciated by those skilled in the art that the choice of the regulatory sequences can depend on factors such as the host cell, the vector and the level of expression desired. The nucleic acid encoding the antigen is operatively linked to a gene expression sequence which directs the expression of the antigen nucleic acid within a eukaryotic cell. The gene expression sequence is any regulatory nucleotide sequence, such as a promoter sequence or promoter-enhancer combination, which facilitates the efficient transcription and translation of the antigen nucleic acid to which it is operatively linked. The gene expression sequence may, for example, be a mammalian or viral promoter, such as a constitutive or inducible promoter. Constitutive mammalian promoters include, but are not limited to, the promoters for the following genes: hypoxanthine phosphoribosyl transferase (HPRT), adenosine deaminase, pyruvate kinase, b-actin promoter and other constitutive promoters. Exemplary viral promoters which function constitutively in eukaryotic cells include, for example, promoters from the cytomegalovirus (CMV), simian virus (e.g., SV40), papilloma virus, adenovirus, human immunodeficiency virus (HIV), Rous sarcoma virus, cytomegalovirus, the long terminal repeats (LTR) of Moloney leukemia virus and other retroviruses, and the thymidine kinase promoter of herpes simplex virus. Other constitutive promoters are known to those of ordinary skill in the art. The promoters useful as gene expression sequences of the invention also include inducible promoters. Inducible promoters are expressed in the presence of an inducing agent. For example, the metallothionein promoter is induced to promote transcription and translation in the presence of certain metal ions. Other inducible promoters are known to those of ordinary skill in the art. In general, the gene expression sequence shall include, as necessary, 5' non-transcribing and 5' non-translating sequences involved with the initiation of transcription and translation, respectively, such as a TATA box, capping sequence, CAAT sequence, and the like. Especially, such 5' non-transcribing sequences will include a promoter region which includes a promoter sequence for transcriptional control of the operably joined antigen nucleic acid. The gene expression sequences optionally include enhancer sequences or upstream activator sequences as desired. Preferred promoters for use in a poxviral vector (see below) include without limitation vaccinia promoters 7.5K, H5R, TK, p28, p11 and K1L, chimeric promoters between early and late poxviral promoters as well as synthetic promoters such as those described in Chakrabarti et al. (1997, Biotechniques 23, 1094-1097), Hammond et al. (1997, J. Virological Methods 66, 135-138) and Kumar and Boyle (1990, Virology 179, 151-158).

The promoter is of special importance and the present invention encompasses the use of constitutive promoters which direct expression of the nucleic acid in many types of host cells and those which direct expression only in certain host cells or in response to specific events or exogenous factors (e.g. by temperature, nutrient additive, hormone or other ligand). Suitable promoters are widely described in literature and one may cite more specifically viral promoters such as RSV, SV40, CMV and MLP promoters. Preferred promoters for use in a poxviral vector include without limitation vaccinia promoters 7.5K, H5R, TK, p28, p11 and K1L, chimeric promoters between early and late poxviral promoters as well as synthetic promoters such as those described in Chakrabarti et al. (1997, Biotechniques 23, 1094-1097), Hammond et al. (1997, J. Virological Methods 66, 135-138) and Kumar and Boyle (1990, Virology 179, 151-158).

Those skilled in the art will appreciate that the regulatory elements controlling the expression of the nucleic acid molecule of the invention may further comprise additional elements for proper initiation, regulation and/or termination of transcription (e.g. polyA transcription termination sequences), mRNA transport (e.g. nuclear localization signal sequences), processing (e.g. splicing signals), and stability (e.g. introns and non-coding 5' and 3' sequences), translation (e.g. peptide signal, propeptide, tripartite leader sequences, ribosome binding sites, Shine-Dalgamo sequences, etc.) into the host cell or organism.

Alternatively, the recombinant vector in use in the present invention can further comprise at least one nucleic acid encoding at least one cytokine. Suitable cytokines include without limitation interleukins (e.g. IL-2, IL-7, IL-15, IL-18, IL-21) and interferons (e.g. IFNγ, INFα), with a special preference for interleukin IL-2. When the recombinant vaccine of the invention comprises a cytokine-expressing nucleic acid, said nucleic acid may be carried by the recombinant vector encoding the one or more antigen(s) or by an independent recombinant vector which can be of the same or a different origin.

According to one preferred embodiment, the recombinant vector in use in the present invention is encoding all or part of the MUC1 antigen and at least one cytokines above listed, and preferably an interleukin, especially IL2.

Infectious viral particles comprising the above-described recombinant viral vector can be produced by routine process. An exemplary process comprises the steps of:
a. introducing the viral vector into a suitable cell line,
b. culturing said cell line under suitable conditions so as to allow the production of said infectious viral particle,
c. recovering the produced infectious viral particle from the culture of said cell line, and
d. optionally purifying said recovered infectious viral particle.

Cells appropriate for propagating adenoviral vectors are for example 293 cells, PERC6 cells, HER96 cells, or cells as disclosed in WO 94/28152, WO 97/00326, US 6,127,175.

Cells appropriate for propagating poxvirus vectors are avian cells, and most preferably primary chicken embryo fibroblasts (CEF) prepared from chicken embryos obtained from fertilized eggs.

The infectious viral particles may be recovered from the culture supernatant or from the cells after lysis (e.g. by chemical means, freezing/thawing, osmotic shock, mecanic shock, sonication and the like). The viral particles can be isolated by consecutive rounds of plaque purification and then purified using the techniques of the art (chromatographic methods, ultracentrifugation on caesium chloride or sucrose gradient).

If desired, the method or use for treating a patient for human disease human disease (i.e. by administering an immunogenic composition comprising at least one antigen) can be carried out in the selected patients in conjunction with one or more conventional therapeutic modalities (e.g. radiation, chemotherapy and/or surgery). The use of multiple therapeutic approaches provides the selected patient with a broader based intervention. In one embodiment, the method or use for treating a patient for human disease human disease can be preceded or followed by a surgical intervention. In another embodiment, it can be preceded or followed by radiotherapy (e.g. gamma radiation). Those skilled in the art can readily formulate appropriate radiation therapy protocols and parameters which can be used (see for example Perez and Brady, 1992, Principles and Practice of Radiation Oncology, 2nd Ed. JB Lippincott Co; using appropriate adaptations and modifications as will be readily apparent to those skilled in the field). In still another embodiment, the method or use of the invention is associated to chemotherapy with one or more drugs (e.g. drugs which are conventionally used for treating or preventing viral infections, virus-associated pathologic conditions, cancer, and the like).
The patient tested in a method of the present Invention may be a cancer patient which is undergoing chemotherapeutic treatment with a chemotherapeutic agent.

According to one embodiment, the patient receives said chemotherapeutic agent before administration of said immunogenic composition.

According to another embodiment, the patient receives said chemotherapeutic agent after administration of said immunogenic composition.

According to another embodiment, the patient receives said chemotherapeutic agent concomitantly with administration of said immunogenic composition.

Preferred chemotherapeutic agent or cytotoxic drugs include, e.g., Vincristine, .Cisplatin, Azaguanine, Etoposide, Adriamycin, Aclarubicin, Mitoxantrone, Mitomycin, Paclitaxel, Gemcitabine, Taxotere, Dexamethasone, Ara-C, Methylpredrdsolone, Methotrexate, Bleomycin, Methyl-GAG, Carboplatin, 5-FU (5-Fluorouracil), MABTHERA™ (Rituximab), radiation, histone deacetylase (HDAC) inhibitors, and 5-Aza-2'-deoxycytidine (Decitabine).

Exemplary radioactive chemotherapeutic agents include compounds containing alpha emitters such as astatine-21 1, bismuth-212, bismuth-213, lead-212, radium-223, actinium-225, and thorium-227, beta emitters such as tritium, strontium-90, cesium-137, carbon- 11, nitrogen- 13, oxygen-15, fluorine-18, iron-52, cobalt-55, cobalt-60, copper-61, copper-62, copper-64, zinc-62, zinc-63, arsenic-70, arsenic-71, arsenic-74, bromine-76, bromine-79, rubidium-82, yttrium-86, zirconium-89, indium-1 10, iodine-120, iodine-124, iodine-129, iodine-131, iodine-125, xenon- 122, technetium-94m, technetium-94, technetium-99m, and technetium-99, or gamma emitters such as cobalt-60, cesium-137, and technetium-99m. Exemplary chemotherapeutic agents also include antibodies such as Alemtuzumab, Daclizumab, Rituximab (MABTHERA™), Trastuzumab (HERCEPTIN™), Gemtuzumab, Ibrirumomab, Edrecolomab, Tositumomab, CeaVac, Epratuzumab, Mitumomab, Bevacizumab, Cetuximab, Edrecolomab, Lintuzumab, MDX-210, IGN-101, MDX-010, MAb, AME ABX-EGF, EMD 72 000, Apolizumab, Labetuzumab, ior-tl, MDX-220, MRA, H-1 1 scFv, Oregovomab, huJ591 MAb, BZL Visilizumab. TriGem, TriAb, R3, MT-201, G-250, unconjugated, ACA-125, Onyvax-105, CDP- 860, BrevaRex MAb AR54, IMC-ICl 1, GlioMAb-H, ING-I, Anti-LCG MAbs, MT-103, KSB- 303, Therex, KW-2871, Anti-HMI.24, Anti-PTHrP, 2C4 antibody, SGN-30, TRAIL-RI MAb CAT, Prostate cancer antibody, H22xKi-4, ABX-MAl, Imuteran, and Monopharm-C.

Exemplary chemotherapeutic agents also include Acivicin; Aclarubicin; Acodazole Hydrochloride; Acronine; Adozelesin; Adriamycin; Aldesleukin; Altretarnine; Ambomycin; Ametantrone Acetate; Arnmoglutethimide; Amsacrine; Anastrozole; Anthramycin; Asparaginase; Asperlin; Azacitidine; Azetepa; Azotomycin; Batimastat; Benzodepa; Bicalutamide; Bisantrene Hydrochloride; Bisnafide Dimesylate; Bizelesin; Bleomycin Sulfate; Brequinar Sodium; Bropirimine; Busulfan; Cactinomycin; Calusterone; Camptothecin; Caracemide; Carbetimer; Carboplatin; Carmustine; Carubicin Hydrochloride; Carzelesin; Cedefingol; Chlorambucil; Cirolemycin; Cisplatin; Cladribine; Combretestatin A-4; Grisnatol Mesylate; Cyclophosphamide; Cytarabine; Dacarbazine; DACA (N- [2-(Dimethyl-amino) ethyl] acridine-4-carboxamide); Dactinomycin; Daunorubicin Hydrochloride; Daunomycin;. Decitabine; Dexormaplatin; Dezaguanine; Dezaguanine Mesylate; Diaziquone; Docetaxel; Dolasatins; Doxorubicin; Doxorubicin Hydrochloride; Droloxifene; Droloxifene Citrate; Dromostanolone Propionate; Duazomycin; Edatrexate; Eflornithine Hydrochloride; Ellipticine; Elsamitrucin; Enloplatin; Enpromate; Epipropidine; Epirubicin Hydrochloride; Erbulozole; Esorubicin Hydrochloride; Estramustine; Estramustine Phosphate Sodium; Etanidazole; Ethiodized Oil 1 131; Etoposide; Etoposide Phosphate; Etoprine; Fadrozole Hydrochloride; Fazarabine; Fenretinide; Floxuridine; Fludarabine Phosphate; Fluorouracil; 5-FdUMP; Flurocitabine; Fosquidone; Fostriecin Sodium; Gemcitabine; Gemcitabine Hydrochloride; Gold Au 198; Homocamptothecin; Hydroxyurea; Idarubicin Hydrochloride; Ifosfamide; llmofosine; Interferon Alfa-2a; Interferon Alfa-2b; hiterferon Alfa-nl; Interferon Alfa-n3; Interferon Beta-I a; Interferon Gamma-I b; Iproplatin; Irinotecan Hydrochloride; Lanreotide Acetate; Letrozole; Leuprolide Acetate; Lïarozole Hydrochloride; Lometrexol Sodium; Lomustine; Losoxantrone Hydrochloride; Masoprocol; Maytansine; Mechlorethamine Hydrochloride; Megestrol Acetate; Melengestrol Acetate; Melphalan; Menogaril; Mercaptopurine; Methotrexate; Methotrexate Sodium; Metoprine; Meturedepa; Mitindomide; Mitocarcin; Mitocromin; Mitogillin; Mitomalcin; Mitomycin; Mitosper; Mitotane; Mitoxantrone Hydrochloride; Mycophenolic Acid; Nocodazole; Nogalamycin;Ormaplatin; Oxisuran; Paclitaxel; Pegaspargase; Peliomycin; Pentamustine; PeploycinSulfate; Perfosfamide; Pipobroman; Piposulfan; Piroxantrone Hydrochloride; Plicamycin; Plomestane; Porfimer Sodium; Porfiromycin; Prednimustine; Procarbazine Hydrochloride; Puromycin; Puromycin Hydrochloride; Pyrazofurin; Rhizoxin; Rhizoxin D; Riboprine; Rogletimide; Safingol; Safingol Hydrochloride; Semustine; Simtrazene; Sparfosate Sodium; Sparsomycin; Spirogermanium Hydrochloride; Spiromustine; Spiroplatin; Streptonigrin; Streptozocin; Strontium Chloride Sr 89; Sulofenur; Talisomycin; Taxane; Taxoid; Tecogalan Sodium; Tegafur; Teloxantrone Hydrochloride; Temoporfin; Tenyposide; Teroxirone; Testolactone; Thiamiprine; Thioguanine; Thiotepa; Thymitaq; Tiazofurin; Tirapazamine; Tomudex; TOP53; Topotecan Hydrochloride; Toremifene Citrate; Trestolone Acetate; Triciribine Phosphate; Trimetrexate; Trimetrexate Glucuronate; Triptorelin; Tubulozole Hydrochloride; Uracil Mustard; Uredepa; Vapreotide; Verteporfin; Vinblastine; Vinblastine Sulfate; Vincristine; Vincristine Sulfate; Vindesine; Vindesine Sulfate; Vinepidine Sulfate; Vinglycinate Sulfate; Vinleurosine Sulfate; Vinorelbine Tartrate; Vinrosidine Sulfate; Vinzolidine Sulfate; Vorozole; Zeniplatin; Zinostatin; Zorubicin Hydrochloride; 2- Chlorodeoxyadenosine; 2' Deoxyformycin; 9-aminocamptothecin; raltitrexed; N-propargyl-5,8- dideazafolic acid; 2chloro-2'-arabino-fluoro-2'-deoxyadenosine; 2-chloro-2'-deoxyadenosine; anisomycin; trichostatin A; hPRL-G129R; CEP-751; linomide; sulfur mustard; nitrogen mustard (mechlor ethamine); cyclophosphamide; melphalan; chlorambucil; ifosfamide; busulfan; N- methyl-Nnitrosourea (MNU); N, N'-Bis (2-chloroethyl)-N-nitrosourea (BCNU); N- (2- chloroethyl)-N' cyclohexyl-N-nitrosourea (CCNU); N- (2-chloroethyl)-N'-(trans-4-methylcyclohexyl-N-nitrosourea (MeCCNU); N- (2-chloroethyl)-N'- (diethyl) ethylphosphonate- N-nitrosourea (fotemustine); streptozotocin; diacarbazine (DTIC); mitozolomide; temozolomide; tbiotepa; mitomycin C; AZQ; adozelesin; Cisplatin; Carboplatin; Ormaplatin; Oxaliplatin;Cl- 973; DWA 2 114R; JM216; JM335; Bis (platinum); tomudex; azacitidine; cytarabine; gemcitabine; 6-Mercaptopurine; 6-Thioguanine; Hypoxanthine; teniposide 9-amino camptothecin; Topotecan; CPT-Il; Doxorubicin; Daunomycin; Epirubicin; darubicin; mitoxantrone; losoxantrone; Dactinomycin (Actinomycin D); amsacrine; pyrazoloacridine; all- trans retinol; 14-hydroxy-retro-retinol; all-trans retinoic acid; N- (4- Hydroxyphenyl) retinamide; 13-cis retinoic acid; 3-Methyl TTNEB; 9-cis retinoic acid; fludarabine (2-F-ara-AMP); or 2-chlorodeoxyadenosine (2-Cda).

Other chemotherapeutic agents include, but are not limited to, 20-pi-l,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; argininedeaminase; asulacrine; atamestane; atrimustine; axinastatin 1; axinastatin 2; axinastatin 3; azasetron; azatoxin; azatyrosine; baccatin DI derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bleomycin A2; bleomycin B2; bropirimine; budotitane; buthionine sulfoximine; calcipotriol; calphostin C; camptothecin derivatives (e.g., 10-hydroxy-camptothecin); canarypox HL-2; capecitabine; carboxamide-amino-triazole; carboxyamidotriazole; CaRestM3; CARN 700; cartilage derived inhibitor; carzelesin; casein kinase inhibitors (ICOS); castanospermine; cecropin B; cetrorelix; chlorins; chloroquinoxaline sulfonamide; cicaprost; cis-porphyrin; cladribine; clomifene analogues; clotrimazole; collismycin A ; collismycin B; combretastatin A4; combretastatin analogue; conagenin; crambescidin 816 ; crisnatol; cryptophycin 8; cryptophycin A derivatives; curacin A ; cyclopentanthraquinones; cycloplatam; cypemycin; cytarabine ocfosfate; cytolytic factor; cytostatin; dacliximab; decitabine; dehydrodidemnin B; 2'deoxycoformycin (DCF); deslorelin; dexifosfamide; dexrazoxane; dexverapamii; diaziquone; didernnin B; didox; diethylnorspermine; dihydro-5-azacytidine; dihydrotaxol, 9- ; dioxamycin; diphenyl spiromustine; discodermolide; docosanol; dolasetron; doxifluridine; droloxifene; dronabinol; duocarmycin SA; ebselen; ecomustine; edelfosine; edrecolomab; eflornithine; elemene; ernitefur; epirubicin; epothilones (A, R = H; B R = Me); epithilones; epristeride; estramustine analogue; estrogen agonists; estrogen antagonists; etanidazole; etoposide; etoposide 4'-phosphate (etopofos); exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; homoharringtonine (HHT); hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4- ; irinotecan; iroplact; irsogladine; isobengazole; isohomohalicondrin B; itasetron; jasplakinolide; kahalalide F; lamellarin-N triacetate; lanreotide; leinamycin; lenograstim; lentinan sulfate; Ieptolstatin; letrozole; leukemia inhibiting factor; leukocyte alpha interferon; leuprolide + estrogen progesterone; leuprorelin; levamisole; liarozole; linear polyamine analogue; lipophilic disaccharide peptide; lipophilic platinum compounds; lissoclïnamide 7; lobaplatin; lombricine; lometrexol; lonidamine; losoxantrone; lovastatin; loxoribine; lurtotecan; lutetium texaphyrin; lysofylline; lytic peptides; maytansine; mannostatin A; marimastat; masoprocol; maspin; matrilysi αinhibitors; matrix metalloproteinase inhibitors; menogaril; rnerbarone; meterelin; methioninase; metoclopramide; IvHF inhibitor; ifepristone; miltefosine; mirimostim; mismatched double stranded RNA; mithracin; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A + myobacteriurn cell wall sk; mopidamol; multiple drug resistance gene inhibitor; multiple tumor suppressor 1- based therapy; mustard anticancer agent; mycaperoxide B; mycobacterial cell wall extract; myriaporone; N-acetyldinaline; N-substituted benzamides; nafarelin; nagrestip; naloxone + pentazocine; napavin; naphterpin; nartograstim; nedaplatin; nemorubicin; neridronic acid; neutral endopeptidase; nilutamide; nisamycin; nitric oxide modulators; nitroxide antioxidant; nitruUyn; 06-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; podophyllotoxin; porfirner sodium; porfiromycin; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; roMtukine; romurtide; roquinimex; rubiginone B 1; ruboxyl; safïngol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfïn; temozolomide; teniposide; tetrachlorodecaoxide; tetrazomine; thaliblastine; thalidomide; thiocoraline; thrombopoietin; thrombopoietin mimetic; thyrnalfasin; thymopoietin receptor agonist; thymotrinan; thyroid stimulating hormone; tin ethyl etiopurpurin; tirapazamine; titanocene dichloride; topotecan; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus- derived growth inhibitory factor; urokinase receptor antagonists; vapreotide; variolin B; vector system, erythrocyte gene therapy; velaresol; veramine; verdins; verteporfln; vinorelbine; vinxaltine; vitaxin; vorozole; zanoterone; zeniplatin; zilascorb; and zinostatin stimalamer.

In another embodiment, the patient tested according to a method or use of the invention is treated with a prime boost therapeutic modality which comprises sequential administration of one or more primer composition(s) and one or more booster composition(s). Typically, the priming and the boosting compositions use different vehicles which comprise or encode at least an antigenic domain in common. The priming composition is initially administered to the host organism and the boosting composition is subsequently administered to the same host organism after a period varying from one day to twelve months.

Such a method may comprise one to ten sequential administrations of the priming composition followed by one to ten sequential administrations of the boosting composition. Desirably, injection intervals are a matter of one week to six months. Moreover, the priming and boosting compositions can be administered at the same site or at alternative sites by the same route or by different routes of administration.

According to one special embodiment, the Invention relates to a method as above described wherein said human disease is cancer.

According to a special embodiment, said cancer is for example breast cancer, colon cancer, kidney cancer, rectal cancer, lung cancer, cancer of the head and neck, renal cancer, malignant melanoma, laryngeal cancer, ovarian cancer, cervical cancer, prostate cancer, non Small cell Lung Cancer (NSCLC), haematological cancers, gastric cancers, myeloma.

According to a preferred embodiment, said cancer is non Small cell Lung Cancer (NSCLC).

According to one special embodiment, the Invention relates to a method as above described wherein said human disease is infectious disease.

According to a preferred embodiment, said infectious disease is a viral induced disease, such as for example disease induced by HIV, HCV, HBV, HPV, and the like.

As described above, the method according to the present invention allows to predict whether a patient will develop a prophylactic or therapeutic response, preferably an immune response towards the administered immunogenic composition.

According to one special embodiment, said "raised immune response" in said patient population is directed towards a tumour-specific or -related antigens and/or viral antigen. According to one embodiment, said "raised immune response" in said patient population is directed towards distinct antigens. According to one special embodiment, said "raised immune response" in said patient population is directed towards MUC1 antigen. According to another special embodiment, said "raised immune response" in said patient population is T cell immune response, and preferably CD8+ (Cytotoxic T Lymphocytes) immune response. According to another special embodiment, said "raised immune response" in said patient population is a non specific immune response. According to another special embodiment, said "raised immune response" in said patient population is a stimulation of the innate immune response.

The ability to induce or stimulate an immune response upon administration in an animal or human organism can be evaluated either in *vitro* or *in vivo* using a variety of assays which are standard in the art. For a general description of techniques available to evaluate the onset and activation of an immune response, see for example Coligan et al. (1992 and 1994, Current Protocols in Immunology ; ed J Wiley & Sons Inc, National Institute of Health). Measurement of cellular immunity can be performed by measurement of cytokine profiles secreted by activated effector cells including those derived from CD4+ and CD8+ T-cells (e.g. quantification of IL-10 or IFN gamma-producing cells by ELIspot), by determination of the activation status of immune effector cells (e.g. T cell proliferation assays by a classical [³H] thymidine uptake), by assaying for antigen-specific T lymphocytes in a sensitized subject (e.g. peptide-specific lysis in a cytotoxicity assay) or by detection of antigen specific T cells by fluorescent MHC and/or peptide multimers (e.g. tetramers). The ability to stimulate a humoral response may be determined by antibody binding and/or competition in binding (see for example Harlow, 1989, Antibodies, Cold Spring Harbor Press). The method of the invention can also be further validated in animal models challenged with an appropriate tumor-inducing agent (e.g. MUCl-expressing TCl cells) to determine anti-tumor activity, reflecting an induction or an enhancement of an anti-antigen immune response.

Thus, the method of the present invention can be used to determine whether the survival of a patient treated for human disease by administering an immunogenic composition can be extended.

According to another embodiment, the present Invention relates to the use of sICAM-1 in a blood sample as a biomarker for predicting whether a subject is or is not susceptible to developing prophylactic or therapeutic response, preferably a prophylactic or therapeutic immune response, by administration of an immunogenic composition, wherein said blood sample is obtained from said patient before administration of the immunogenic composition and wherein said immunogenic composition contains all or part of at least one targeted antigen or at least one recombinant vector expressing in vivo all or part of at least one heterologous nucleotide sequence encoding all or part of at least one targeted antigen.

More specifically, the present Invention relates to the use of the level of sICAM-1 in a blood sample as a biomarker for predicting whether a subject is or is not susceptible to developing prophylactic or therapeutic response, preferably prophylactic or therapeutic immune response, by administration of an immunogenic composition as defined above, wherein low levels of sICAM-1 indicates that the subject is predicted to have an increased susceptibility to develop a prophylactic or therapeutic response, preferably prophylactic or therapeutic immune response.

In other words, the present Invention relates to the use of the level of sICAM-1 in a blood sample as a biomarker for predicting whether a subject is or is not susceptible to survive longer after administration of an immunogenic composition as defined above, wherein low levels of sICAM-1 indicates that the subject is predicted to have a longer survival rate compared to treated patients who have higher levels of sICAM-1.

Also described are kits which include parts for practicing the methods described herein and that will be apparent from the examples provided herein. The kit of parts, or kits, may include reagents for collecting and or measuring total blood, serum or plasma levels of sICAM-1. Such reagents may include antibodies. The kits may further include equipment for collecting and/or processing biological samples. The kits are also likely to contain instructions for use, cut-off values and/or instructions for their determination, and instructions for interpreting the data obtained from the use of the kits.

Such a kit of parts, or kits, may further include an immunogenic composition as above disclosed, and/or as disclosed in the Example section below.

Also disclosed are computer programs and/or algorithms for monitoring clinical trial and sICAM-1levels, determining whether such levels are above or below a threshold level, and/or recommending modifications to a treatment regiment to improve a patient's response to an immunotherapy treatment. The computer programs or algorithms may be provided along with necessary hardware, e.g., in the form of a kit or apparatus, which may also accept biological samples and measure the relative levels of sICAM-1 present therein. The above-described computer programs and/or apparatus are likely to be provided to physicians or clinical laboratories with appropriate instructions and reagents, including antibodies.

The invention has been described in an illustrative manner, and it is to be understood that the terminology which has been used is intended to be in the nature of words of description rather than of limitation. Obviously, many modifications and variations of the present invention are possible in light of the above teachings. It is therefore to be understood that within the scope of the appended claims, the invention may be practiced in a different way from what is specifically described herein.

### EXAMPLES

### Figures :

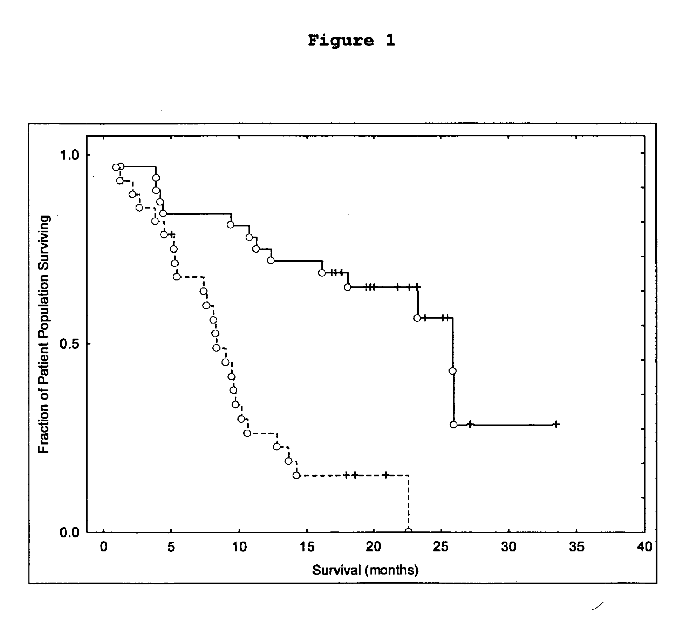

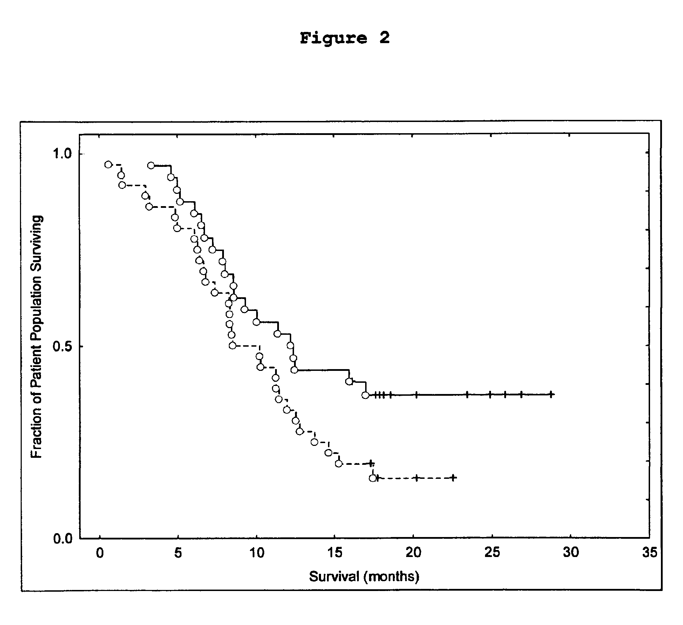

**Figure 1****: Survival curves describing vaccine immunotherapy in lung cancer: patients with ≤ or > 224 ng/ml sCD54**
   - Group 1 : Vaccine (i.e. immunogenic composition) + chemotherapy in patients with low levels of sCD54. Low levels as defined as ≤ 224 ng/ml sCD54 in peripheral blood plasma. 32 patients.
   - - - - - Group 2 : Vaccine (i.e. immunogenic composition) + chemotherapy in patients with high levels of sCD54. High levels as defined as > 224 ng/ml sCD54 in peripheral blood plasma. 29 patients.
   Significant difference, by log rank: p = 0.00006
   ○ Complete + Censored
**Figure 2****: Survival curves describing chemotherapy in lung cancer: patients with ≤ or > 224 ng/ml sCD54**
   - Group 1 : Chemotherapy alone (without vaccine) in patients with low levels of sCD54. Low levels as defined as ≤ 224 ng/ml sCD54 in peripheral blood plasma. 32 patients.
   - - - - - Group 2 : Chemotherapy alone (without vaccine) in patients with high levels of sCD54. High levels as defined as > 224 ng/ml sCD54 in peripheral blood plasma. 36 patients.
   Significant difference, by log rank: p = 0.064 (not statistically significant)
   ○ Complete + Censored

### Example 1 :

The immunogenic composition, noted vaccine TG4010, was used to treat non-small cell lung cancer (NSCLC) patients in combination with standard chemotherapy.

TG4010 is a recombinant Modified Virus Ankara (MVA) expressing both IL2 and the tumor-associated antigen MUC1.

One hundred and forty eight patients were randomized to receive:
- chemotherapy (Cisplatin 75mg/m² on d1 and Gemcitabine 1250mg/m² on day 1 and day 8 every 3 weeks for up to 6 cycles) either alone (Study Arm 2) or
- chemotherapy together with TG4010 (-Study Arm 1).

Tumors were evaluated (WHO criteria) every 6 weeks. Endpoints were progression-free survival (PFS) at 6 months and overall survival with intent to treat analysis.

Blood samples were taken prior to treatment and were shipped immediately to a central immunology lab where plasma samples were aliquoted and frozen. Frozen aliquots of plasma were shipped, in batch, on dry ice, to a second central lab where sCD54 levels were assessed.

Plasma samples were assessed for content of sCD54 (sICAM-1) by Multi-analyte plasma protein profiling using the Luminex® system. The limit of 224 ng/ml was the median value for all 129 patient baseline plasma samples analysed.

Figure 1 shows that patients [Arm 1 (TG4010 + chemotherapy)] with **≤** 224 ng/ml sCD54 (ICAM1) at baseline survive longer (median survival = 24.5 months) than do patients with > 224 ng/ml sCD54 (ICAM-1) (median survival 8.4 months) when treated with both the TG4010 vaccine and chemotherapy.

The data in Figure 2 demonstrate that the effect of selecting patients based on the plasma content of sCD54 (sICAM-1) is restricted to patients receiving the vaccine. Figure 2, illustrating the use of conventional chemotherapy alone (Cisplatin 75mg/m² on d1 and Gemcitabine 1250mg/m² on day 1 and day 8 every 3 weeks for up to 6 cycles),shows that patients with **≤** or > 224 ng/ml sCD54 (ICAM-1) at baseline have not significantly different survival expectancy. Median survival of 12.2 months and 8.5 months for ≤ and > 224 ng/ml sCD54 (ICAM-1), respectively, were observed.

## Claims

1. An ex-vivo method for testing whether a patient will respond therapeutically to a method of treatment comprising administration of an immunogenic composition, wherein the testing method comprises the step of measuring levels of sICAM-1 in a blood sample obtained from said patient before administration of said immunogenic composition wherein low levels of sICAM-1 indicate that the patient will develop a prophylactic or therapeutic response towards the immunogenic composition with low levels of sICAM-1 being fewer than 300 ng/ml, and wherein said immunogenic composition contains all or part of at least one targeted antigen or at least one recombinant vector expressing *in vivo* all or part of at least one heterologous nucleotide sequence encoding all or part of at least one targeted antigen.

2. The method of claim 1 wherein said low levels of sICAM-1 means levels of fewer than 250 ng/ml.

3. The method of claim 1 wherein said low levels of sICAM-1 means levels of fewer than 224 ng/ml.

4. The method of claim 1 wherein said low levels of sICAM-1 means levels of fewer than 200 ng/ml.

5. The method of any one of claims 1 to 4 wherein said method of treatment is a method of treating cancer.

6. The method of any one of the preceding claims, wherein said level of sICAM-1 is measured with multi-analyte plasma protein profiling or enzyme-linked immunosorbant assays.

7. The method of any one of the preceding claims, wherein said level of sICAM-1 is determined by using antibodies.

8. The method of any one of the preceding claims, wherein said blood sample is selected from the group consisting of total blood sample, plasma or serum.

9. The method of any one of claims 1 to 8, wherein said recombinant vector is a viral vector.

10. The method of claim 9, wherein said viral vector is replication-competent.

11. The method of claim 9, wherein said viral vector is replication-defective.

12. The method of any one of claims 9 to 11, wherein said recombinant vector is a recombinant adenoviral vector.

13. The method of any one of claims 9 to 11, wherein said recombinant vector is a recombinant vaccinia vector.

14. The method of claim 13, wherein said recombinant vaccinia vector is a recombinant MVA vector.

15. The method of any one of the preceding claims, wherein said patient is a patient treated with a chemotherapeutic agent.

16. Use of levels of sICAM-1 in a blood sample as a biomarker for predicting whether a patient is or is not susceptible to developing a prophylactic or therapeutic immune response by administration of an immunogenic composition, wherein said blood sample is obtained from said patient before administration of the immunogenic composition and wherein said immunogenic composition contains all or part of at least one targeted antigen or at least one recombinant vector expressing *in vivo* all or part of at least one heterologous nucleotide sequence encoding all or part of at least one targeted antigen.

## Patentansprüche

1. *Ex*-*vivo*-Verfahren zum Testen, ob ein Individuum therapeutisch auf ein Behandlungsverfahren anspricht, umfassend die Verabreichung einer immunogenen Zusammensetzung, wobei das Testverfahren den Schritt des Messens von sICAM-1-Spiegeln in einer Blutprobe umfasst, die von dem Individuum vor der Verabreichung der immunogenen Zusammensetzung erhalten wurde, wobei niedrige Spiegel von sICAM-1 anzeigen, dass der Patient eine prophylaktische oder therapeutische Antwort gegen die immunogene Zusammensetzung entwickeln wird, wobei niedrige Spiegel von sICAM-1 weniger als 300 ng/ml sind, und wobei die immunogene Zusammensetzung mindestens ein Zielantigen ganz oder teilweise oder mindestens einen rekombinanten Vektor enthält, der *in vivo* mindestens eine heterologe Nucleotidsequenz ganz oder teilweise exprimiert, die mindestens ein Zielantigen ganz oder teilweise codiert.

2. Verfahren nach Anspruch 1, wobei die niedrigen Spiegel von sICAM-1-Spiegel von weniger als 250 **ng/ml** bedeuten.

3. Verfahren nach Anspruch 1, wobei die niedrigen Spiegel von sICAM-1-Spiegel von weniger als 224 ng/ml bedeuten.

4. Verfahren nach Anspruch 1, wobei die niedrigen Spiegel von sICAM-1-Spiegel von weniger als 200 ng/ml bedeuten.

5. Verfahren nach einem der Ansprüche 1 bis 4, wobei das Behandlungsverfahren ein Verfahren zur Behandlung von Krebs ist.

6. Verfahren nach einem der vorhergehenden Ansprüche, wobei der Spiegel von sICAM-1 durch Multi-Analyt-Plasma-Protein-Profiling oder Enzyme-linked Immunosorbent Assays gemessen wird.

7. Verfahren nach einem der vorhergehenden Ansprüche, wobei der Spiegel von sICAM-1 durch Verwendung von Antikörpern bestimmt wird.

8. Verfahren nach einem der vorhergehenden Ansprüche, wobei die Blutprobe ausgewählt ist aus der Gruppe bestehend aus einer Gesamtblutprobe, Plasma oder Serum.

9. Verfahren nach einem der Ansprüche 1 bis 8, wobei der rekombinante Vektor ein viraler Vektor ist.

10. Verfahren nach Anspruch 9, wobei der virale Vektor replikationskompetent ist.

11. Verfahren nach Anspruch 9, wobei der virale Vektor replikationsdefekt ist.

12. Verfahren nach einem der Ansprüche 9 bis 11, wobei der rekombinante Vektor ein rekombinanter adenoviraler Vektor ist.

13. Verfahren nach einem der Ansprüche 9 bis 11, wobei der rekombinante Vektor ein rekombinanter Vaccinia-Vektor ist.

14. Verfahren nach Anspruch 13, wobei der rekombinante Vaccinia-Vektor ein rekombinanter MVA-Vektor ist.

15. Verfahren nach einem der vorhergehenden Ansprüche, wobei das Individuum Patient ein Individuum ist, der mit einem chemotherapeutischen Mittel behandelt wird.

16. Verwendung von sICAM-1-Spiegeln in einer Blutprobe als ein Biomarker für die Vorhersage, ob ein Patient empfänglich oder nicht empfänglich für die Entwicklung einer prophylaktischen oder therapeutischen Immunantwort durch Verabreichung einer immunogenen Zusammensetzung ist, wobei die Blutprobe von dem Individuum vor der Verabreichung der immunogenen Zusammensetzung erhalten wurde, und wobei die immunogene Zusammensetzung mindestens ein Zielantigen ganz oder teilweise oder mindestens einen rekombinanten Vektor enthält, der *in vivo* mindestens eine heterologe Nucleotidsequenz ganz oder teilweise exprimiert, die mindestens ein Zielantigen ganz oder teilweise codiert.

## Revendications

1. Méthode *ex vivo* pour tester si un patient répondra thérapeutiquement à une méthode de traitement comprenant l'administration d'une composition immunogène, où la méthode de test comprend l'étape de mesure des taux d'ICAM-1 dans un échantillon de sang obtenu à partir dudit patient avant l'administration de ladite composition immunogène, où des taux bas d'ICAM-1s indiquent que le patient développera une réponse prophylactique ou thérapeutique envers la composition immunogène avec les taux bas d'ICAM-1s étant inférieurs à 300 ng/ml, et où ladite composition immunogène contient la totalité ou une partie d'au moins un antigène ciblé ou d'au moins un vecteur recombinant exprimant *in vivo* la totalité ou une partie d'au moins une séquence nucléotidique hétérologue codant pour la totalité ou une partie d'au moins un antigène ciblé.

2. Méthode selon la revendication 1, dans laquelle lesdits taux bas d'ICAM-1s signifient des taux inférieurs à 250 ng/ml.

3. Méthode selon la revendication 1, dans laquelle lesdits taux bas d'ICAM-1s signifient des taux inférieurs à 224 ng/ml.

4. Méthode selon la revendication 1, dans laquelle lesdits taux bas d'ICAM-1s signifient des taux inférieurs à 200 ng/ml.

5. Méthode selon l'une quelconque des revendications 1 à 4, ladite méthode de traitement étant une méthode de traitement du cancer.

6. Méthode selon l'une quelconque des revendications précédentes, dans laquelle ledit taux d'ICAM-1s est mesuré avec des tests multi-analytes de profilage des protéines plasmatiques ou de dosage d'immunoadsorption par enzyme liée.

7. Méthode selon l'une quelconque des revendications précédentes, dans laquelle ledit taux d'ICAM-1s est déterminé en utilisant des anticorps.

8. Méthode selon l'une quelconque des revendications précédentes, dans laquelle ledit échantillon de sang est choisi dans le groupe constitué d'un échantillon de sang total, de plasma ou de sérum.

9. Méthode selon l'une quelconque des revendications 1 à 8, dans laquelle ledit vecteur recombinant est un vecteur viral.

10. Méthode selon la revendication 9, dans laquelle ledit vecteur viral est compétent pour la réplication.

11. Méthode selon la revendication 9, dans laquelle ledit vecteur viral est défectueux pour la réplication.

12. Méthode selon l'une quelconque des revendications 9 à 11, dans laquelle ledit vecteur recombinant est un vecteur adénoviral recombinant.

13. Méthode selon l'une quelconque des revendications 9 à 11, dans laquelle ledit vecteur recombinant est un vecteur de la vaccine recombinant.

14. Méthode selon la revendication 13, dans laquelle ledit vecteur de la vaccine recombinant est un vecteur MVA recombinant.

15. Méthode selon l'une quelconque des revendications précédentes, dans laquelle ledit patient est un patient traité avec un agent chimiothérapeutique.

16. Utilisation des taux d'ICAM-1s dans un échantillon de sang en tant que biomarqueur pour prédire si un patient est susceptible ou pas de développer une réponse immunitaire prophylactique ou thérapeutique par administration d'une composition immunogène, où ledit échantillon de sang est obtenu à partir dudit patient avant l'administration de la composition immunogène et où ladite composition immunogène contient la totalité ou une partie d'au moins un antigène ciblé ou d'au moins un vecteur recombinant exprimant *in vivo* la totalité ou une partie d'au moins une séquence nucléotidique hétérologue codant pour la totalité ou une partie d'au moins un antigène ciblé.
